(19) Europäisches Patentamt
European Patent Office
Office européen des brevets

(11) Veröffentlichungsnummer: **0 228 607**
**B1**

(12) # EUROPÄISCHE PATENTSCHRIFT

(45) Veröffentlichungstag der Patentschrift: **18.04.90**

(21) Anmeldenummer: **86117009.0**

(22) Anmeldetag: **08.12.86**

(51) Int. Cl.[5]: **C 07 D 249/00,**
C 07 D 233/61,
A 01 N 43/653, A 01 N 43/50

(54) **Substituierte Cyclopropyl-oximether.**

(30) Priorität: **19.12.85 DE 3545085**

(43) Veröffentlichungstag der Anmeldung:
**15.07.87 Patentblatt 87/29**

(45) Bekanntmachung des Hinweises auf die Patenterteilung:
**18.04.90 Patentblatt 90/16**

(84) Benannte Vertragsstaaten:
**AT BE CH DE FR GB IT LI NL**

(56) Entgegenhaltungen:
**EP-A-0 158 299**
**DE-A-2 816 817**

(73) Patentinhaber: **BAYER AG**
**D-5090 Leverkusen 1 Bayerwerk (DE)**

(72) Erfinder: **Knops, Hans-Joachim, Dr.**
**Köpenickerstrasse 35**
**D-4019 Monheim (DE)**
Erfinder: **Steinbeck, Karl, Dr. c/o Mobay Corporation**
**8400 Hawthorn Road P.O. Box 49 13**
**Kansas City, Missouri 64120-0013 (US)**
Erfinder: **Büchel, Karl Heinz, Prof. Dr.**
**Dabringhausener Strasse 42**
**D-5093 Burscheid (DE)**
Erfinder: **Brandes, Wilhelm, Dr.**
**Eichendorffstrasse 3**
**D-5653 Leichlingen 1 (DE)**
Erfinder: **Reinecke, Paul, Dr.**
**Steinstrasse 8**
**D-5090 Leverkusen 3 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 228 607 B1

**Beschreibung**

Die vorliegende Erfindung betrifft neue substituierte Cyclopropyl-oximether, ein Verfahren zu ihrer Herstellung und ihre Verwendung als Fungizide.

Es ist bereits bekannt geworden, daß bestimmte Oximinotriazolyl-ethane und deren Salze gute fungizide Eigenschaften aufweisen (vgl. DE—A—2 816 817). So lassen sich z.B. 1-(2,4-Dichlorphenyl)-1-(2,4-dichlorbenzyl-oximino)-2-(1,2,4-triazol-1-yl)-ethan-Nitrate und 1-(2,4-Dichlorphenyl)-1-(methoximino)-2-(1,2,4-triazol-1-yl)-ethan-Nitrat zur Bekämpfung von Pilzen einsetzen. Die Wirkung dieser Verbindungen ist jedoch, vor allem bei niedrigen Aufwandmengen, nicht immer voll befriedigend.

Es wurden neue substituierte Cyclopropyl-oximether der Formel

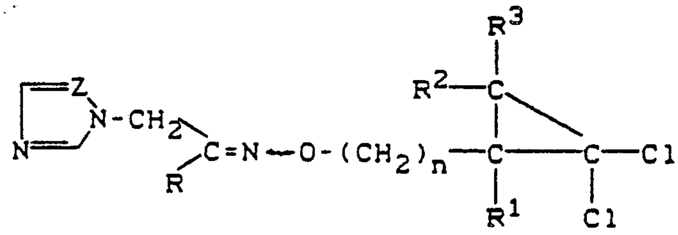

(I)

in welcher
Z für ein Stickstoffatom oder die CH-Gruppe steht,
R für die Gruppierungen

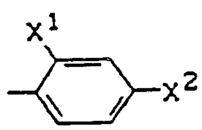

oder

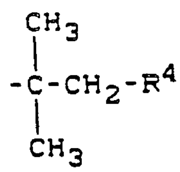

steht,

in welchen
$X^1$ und $X^2$ gleich oder verschieden sind und für Wasserstoff, Halogen oder Halogenalkyl stehen, wobei $X^1$ und $X^2$ nicht gleichzeitig für Wasserstoff stehen,
$R^4$ für Wasserstoff oder gegebenenfalls ein- bis dreifach, gleich oder verschieden substituiertes Phenyl steht, wobei als Substituenten Halogen und Alkyl mit 1 bis 4 Kohlenstoffatomen genannt seien,
$R^1$, $R^2$ und $R^3$ unabhängig voneinander für Wasserstoff, Methyl oder Chlor stehen und
n für ganze Zahlen von 1 bis 6 steht,
sowie deren Säureadditions-Salze und Metallsalz-Komplexe gefunden.

Die Verbindungen der Formel (I) können in der syn- oder anti-Form vorliegen; vorwiegend fallen sie als Gemische beider Formen an.

Weiterhin wurde gefunden, daß man die substituierten Cyclopropyl-oximether der Formel (I) sowie deren Säureadditions-Salze und Metallsalz-Komplexe erhält, wenn man Oxime der Formel

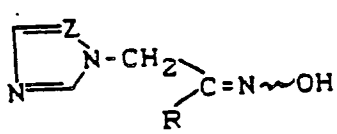

(II)

in welcher
Z und R die oben angegebene Bedeutung haben,
mit Cyclopropan-Derivaten der Formel

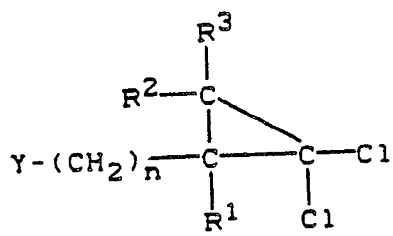

(III)

in welcher
$R^1$, $R^2$, $R^3$ und n die oben angegebene Bedeutung haben und

Y für Halogen oder p-Methyl-phenyl-sulfonyloxy steht, gegebenenfalls in Gegenwart einer starken Base und in Gegenwart eines Verdünnungsmittels umsetzt und gegebenenfalls an die so erhaltenen Verbindungen der Formel (I) eine Säure oder ein Metallsalz addiert.

Schließlich wurde gefunden, daß sich die neuen substituierten Cyclopropyl-oximether der Formel (I) sowie deren Säureadditions-Salze und Metallsalz-Komplexe durch sehr gute fungizide Eigenschaften auszeichnen.

Überraschenderweise besitzen die erfindungsgemäßen Stoffe eine bessere fungizide Wirksamkeit als die aus dem Stand der Technik bekannten Oximino-triazolyl-ethane 1-(2,4-Dichlorphenyl)-1-(2,4-dichlorbenzyloximino)-2-(1,2,4-triazol-1-yl)-ethan-Nitrat und 1-(2,4-Dichlorphenyl)-1-(methoximino)-2-(1,2,4-triazol-1-yl)-ethan-Nitrat, die konstitutionell und wirkungsmäßig naheliegende Verbindungen sind.

Die erfindungsgemäßen substituierten Cyclopropyl-oximether sind durch die Formel (I) allgemein definiert, Bevorzugt sind Verbindungen der Formel (I), in denen

Z für ein Stickstoffatom oder die CH-Gruppe steht,

R für die Gruppierungen

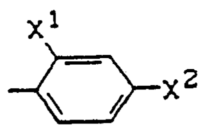 oder 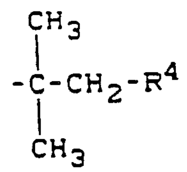 steht,

in welchen

$X^1$ und $X^2$ gleich oder verschieden sind und für Wasserstoff, Fluor, Chlor, Brom oder Halogenalkyl mit 1 oder 2 Kohlenstoffatomen und 1 bis 5 gleichen oder verschiedenen Halogenatomen, wie vorzugsweise Fluor und Chlor stehen, wobei $X^1$ und $X^2$ nicht gleichzeitig für Wasserstoff stehen,

$R^4$ für Wasserstoff oder gegebenenfalls ein- oder zweifach, gleich oder verschieden substituiertes Phenyl steht, wobei als Substituenten Fluor, Chlor oder Methyl genannt seien,

$R^1$, $R^2$ und $R^3$ unabhängig voneinander für Wasserstoff, Methyl oder Chlor stehen und

n für ganze Zahlen von 1 bis 4 steht. Bevorzugte erfindungsgemäße Verbindungen sind auch Additions-Salze aus Säuren und denjenigen substituierten Cyclopropyl-oximethern der Formel (I), in denen Z, R, $R^1$, $R^2$, $R^3$ und n die Bedeutungen haben, die bereits vorzugsweise für diese Substituenten bzw. den Index n genannt wurden.

Zu den Säuren, die addiert werden können, gehöhren vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodsystems der Elemente und denjenigen Verbindungen der Formel (I), in denen Z, R, $R^1$, $R^2$, $R^3$ und n die Bedeutungen haben, die bereits vorzugsweise für diese Substituenten bzw. den Index n genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 1-(2,4-Dichlorphenyl)-1-oximino-2-(1,2,4-triazol-1-yl)-ethan und 2,2-Dichlorcyclopropyl-methylbromid als Ausgangsstoffe, so kann der Reaktionsablauf des erfindungsgemäßen Verfahrens durch das folgende Formelschema wiedergegeben werden:

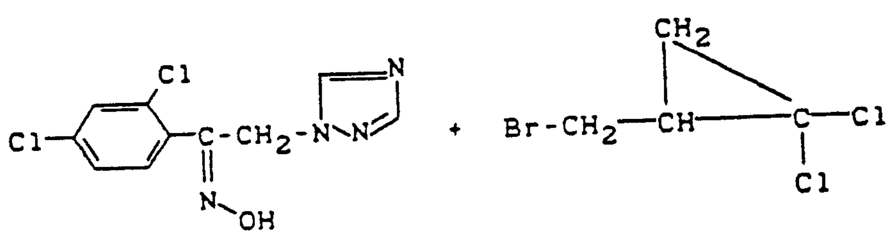

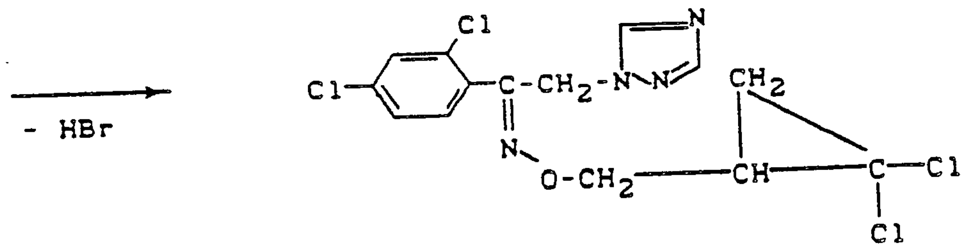


Die bei der Durchführung des erfindungsgemäßen Verfahrens als Ausgangsstoffe benötigten Oxime sind durch die Formel (II) allgemein definiert. In dieser Formel haben Z und R vorzugsweise diejenigen Bedeutungen, die bereits im Zusammenhang mit der Beschreibung der erfindungsgemäßen Stoffe der Formel (I) vorzugsweise für diese Reste genannt wurden.

Die Oxime der Formel (II) sind bekannt (vgl. DE—A—2 431 401, DE—A—2 547 953, DE—A—2 816 817, DE—A—2 824 394 und EP—A—0 094 572. Sie können erhalten werden, indem man Azolylketone der Formel

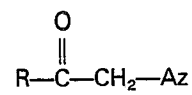
(IV)

in welcher

R die oben angegebene Bedeutung hat und

Az für 1,2,4-Triazol-1-yl oder Imidazol-1-yl steht, mit Hydroxylamin in Gegenwart eines Lösungsmittels, vorzugsweise von Alkoholen bzw. wässrigen Alkoholen, bei Temperaturen zwischen 20°C und 100°C, vorzugsweise zwischen 50°C und 80°C, umsetzt. Dabei wird das Hydroxylamin vorzugsweise in Form seiner Salze, insbesondere als Hydrochlorid, in Gegenwart eines Säurebindemittels, wie beispielsweise Natriumcarbonat, eingesetzt, die Isolierung der Verbindungen der Formel (II) erfolgt dadurch, daß man das während der Umsetzung entstehende Produkt nach Abdestillieren des Lösungsmittels nach üblichen Methoden aufarbeitet.

Die Azolylketone der Formel (IV) können erhalten werden, indem man Halogenketone der Formel

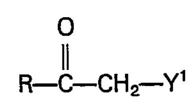
(V)

in welcher

R die oben angegebene Bedeutung hat und

$Y^1$ für Chlor oder Brom steht,

mit 1,2,4-Triazol oder Imidazol in Gegenwart eines Verdünnungsmittels, wie Methylethylketon, und in Gegenwart eines Säurebindemittels, wie Kaliumcarbonat, bei Temperaturen zwischen 20°C und 150°C, vorzugsweise zwischen 60°C und 120°C, umsetzt.

Die außerdem für das erfindungsgemäße Verfahren als Ausgangsstoffe zu verwendenden Cyclopropan-Derivate sind durch die Formel (III) allgemein definiert. In dieser Formel haben $R^1$, $R^2$, $R^3$ und n vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. Y steht vorzugsweise für Chlor, Brom oder p-Methyl-phenylsulfonyloxy (=Tosyl).

Die Cyclopropan-Derivate der Formel (III) sind teilweise bekannt (vgl. Liebigs Ann. Chem. *1979*, S. 920 und Synthesis *1974*, s. 274). Sie lassen sich nach prinzipiell bekannten Verfahren synthetisieren. So erhält man Verbindungen der Formel (III) dadurch, daß man Allyl-Derivate der Formel

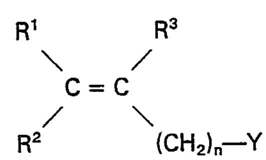
(VI)

in welcher

$R^1$, $R^2$, $R^3$, Y und n die oben angegebenen Bedeutungen haben,

mit Chloroform im Gegenwart von Triethylbenzylammoniumchlorid und wäßriger Natronlauge bei Temperaturen zwischen 35°C und 40°C umsetzt.

Die Allyl-Derivate der Formel (VI) sind allgemein bekannte Verbindungen der organischen Chemie.

Für die erfindungsgemäße Umsetzung kommen als Verdünnungsmittel inerte organische Lösungsmittel infrage. Hierzu gehören vorzugsweise Ether, wie Tetrahydrofuran und Dioxan; aromatische

Kohlenwasserstoffe, wie Toluol und Benzol; sowie Hexamethyl-phosphorsäure-triamid, Säureamide, wie Dimethylformamid und Sulfoxide, wie Dimethylsulfoxid.

Die erfindungsgemäße Umsetzung wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyltrimethyl-ammoniumhydroxid oder Dibenzyl-dimethylammonium-hydroxid und Tetraphenylphosphoniumhydroxid oder Methyltriphenylphosphoniumhydroxid.

Die Reaktionstemperaturen können beim erfindungsgemäßen Verfahren in einem größeren Bereich variiert werden. Im allgemeinen arbeitet man zwischen 0°C und 150°C, vorzugsweise bei Raumtemperatur. In einzelnen Fällen ist es vorteilhaft bei der Siedetemperatur des Lösungsmittels, beispielsweise zwischen 60°C und 100°C, zu arbeiten.

Bei der Durchführung des erfindungsgemäßen Verfahrens setzt man auf 1 Mol Oxim der Formel (II) vorzugsweise 1 bis 3 Mol an Cyclopropan-Derivat der Formel (III) ein. Die Isolierung der Endprodukte der Formel (I) erfolgt in bekannter Weise.

In einer bevorzugten Ausführungsform des erfindungsgemäßen Verfahrens wird die Umsetzung in einem Zweiphasensystem, wie beispielsweise wässrige Natron- oder Kalilauge/Toluol, unter Zusatz von 0,01—1 Mol eines Phasentransferkatalysators, wie beispielsweise Ammonium- oder Phosphoniumverbindungen, durchgeführt.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemäßen Stoffe als bevozugt zu addierende Säuren genannt wurden.

Die Säureadditions-Salze der Verbindungen der Formel (I) lassen sich in einfacher Weise nach üblichen Salzbildungsmethoden herstellen. Im allgemeinen verfährt man so, daß man eine Verbindung der Formel (I) in einem geeigneten inerten Verdünnungsmittel löst und dann eine Säure hinzufügt. Die Isolierung erfolgt in bekannter Weise, zum Beispiel dadurch, daß man das Salz abfiltriert und gegebenenfalls durch Waschen mit einem inerten organischen Lösungsmittel reinigt.

Zur Herstellung von Metallsalz-Komplexen der Verbindungen der Formel (I) kommen vorzugsweise Salze von denjenigen Metallen in Betracht, die bereits im Zusammenhang mit der Beschreibung der erfindungsgemäßen Stoffe als bevorzugt zu addierende Metallsalze genannt wurden.

Die Metallsalz-Komplexe von Verbindungen der Formel (I) lassen sich in einfacher Weise nach üblichen Methoden herstellen. Im allgemeinen geht man so vor, daß man ein Metallsalz in Alkohol, wie zum Beispiel Ethanol, löst und dann eine Verbindung der Formel (I) hinzufügt. Die Isolierung erfolgt ebenfalls in bekannter Weise, zum Beispiel dadurch, daß man den Metallsalz-Komplex abfiltriert und gegebenenfalls durch Umkristallisation reinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Asomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseuduoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Spaerotheca fuliginea;
Podosphaera-Arten, wie Podosphaera leucotriocha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helmonthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;

Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Sphaerotheca-Arten, wie Sphaerotheca fuliginea an Gurken, sowie Uromyces-Arten, wie Uromyces appendiculatus, eingesetzt werden. Ferner eignen sich die erfindungsgemäßen Wirkstoffe sehr gut zur Bekämpfung von Venturia-Arten, wie Venturia inaequalis an Äpfeln, sowie Getreidekrankheiten, wie Echter Mehltau, Fusarium, Septoria nodorum, Cochliobolus sativus und Pyrenophora teres.

Die Wirkstoffe können in die üblichen Formulierungen übergeführt werden, die Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate komkmen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemäßen Wirkstoffe geht aus den folgenden Beispielen hervor.

Herstellungsbeispiel:

Beispiel 1

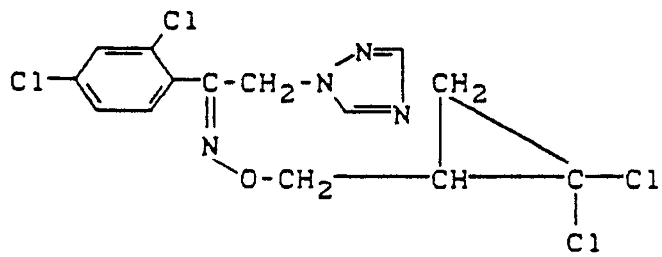

21,4 g (0,078 Mol) 1-(2,4-Dichlorphenyl)-1-oximino-2-(1,2,4-triazol-1-yl)-ethan werden mit 10,8 g (0,078 Mol) Kaliumcarbonat sowie 100 ml Dimethylformamid gemischt, auf 60°C erwärmt und unter Rühren zu einer Lösung von 15,8 g (0,078 Mol) 2,2-Dichlorcyclopropyl-methylbromid in 50 ml Dimethylformamid hinzugetropft. Es wird weitere 12 Stunden bei 60°C gerührt. Das Reaktionsgemisch wird eingeengt. Nach dem Verrühren des Rückstandes mit Wasser wird dreimal mit Methylenchlorid extrahiert. Die organische Phase wird mit Wasser gewaschen, getrocknet und eingeengt. Der verbleibende Rückstand wird chromatographisch über Kieselgel mit Chloroform als Laufmittel gereinigt.

Man erhält 13 g (43% der Theorie) 1-(2,2-Dichlorcyclopropyl-methoximino)-1-(2,4-dichlorphenyl)-2-(1,2,4-triazol-1-yl)-ethan vom Brechnungsindex $n_D^{22} = 1,5647$.

Herstellung der Ausgangsprodukte

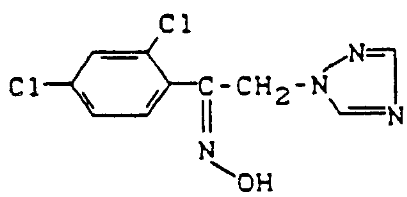

106,8 g (0.44 Mol) 1-(2,4-Dichlorphenyl)-2-(1,2,4-triazol-1-yl)-1-ethanon werden in 780 ml Ethanol gelöst, mit 48 g Hydroxylammoniumhydrochlorid versetzt und 5 Stunden unter Rückfluß erhitzt. Danach wird das Reaktionsgemisch mit 1000 ml Wasser versetzt und filtriert. Man erhält 51 g (45% der Theorie) 1-(2,4-Dichlorphenyl)-1-oximino-2-(1,2,4-triazol-1-yl)-ethan vom Schmelzpunkt 165—170°C.

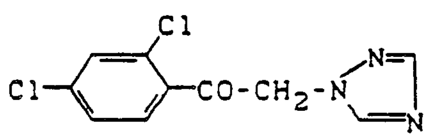

269 g (1 Mol) ω-Brom-2,4-dichloracetophenon werden in 250 ml Acetonitril gelöst. Diese Lösung tropft man zu einer unter Rückfluß siedenden Suspension von 69 g (1 Mol) 1,2,4-Triazol und 150 g Kaliumcarbonat in 2 l Acetonitril. Nach 20-stündigem Erhitzen unter Rückfluß wird die erkaltete Suspension filtriert, das Filtrat vom Lösungsmittel befreit und der Rückstand mit Essigester aufgenommen. Dann wird mit Wasser gewaschen, über Natriumsulfat getrocknet und unter vermindertem Druck vom Lösungsmittel befreit. Der Rückstand kristallisiert beim Versetzen mit Isopropanol aus. Nach dem Umkristallisieren aus Ligroin/Isopropanol erhält man 154 g (60% der Theorie) 1-(2,4-Dichlorphenyl)-2-(1,2,4-triazol-1-yl)-1-ethanon vom Schmelzpunkt 117°C.

In entsprechender Weise und gemäß dem angegebenen Verfahren werden die folgenden Verbindungen der Formel

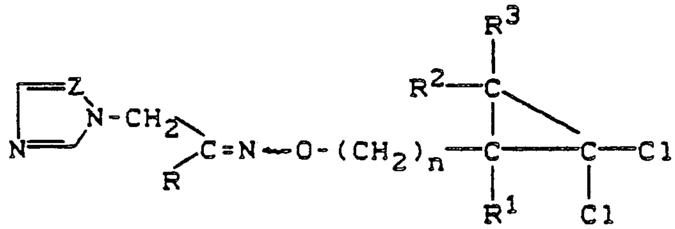

(I)

erhalten:

| Bsp. Nr. | Z | R | n | $R^1$ | $R^2$ | $R^3$ | Physikalische Daten |
|---|---|---|---|---|---|---|---|
| 2 | N | 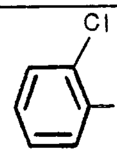 | 1 | H | H | H | $n_D^{22}$ = 1,5707 |
| 3 | N | 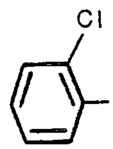 | 1 | H | $CH_3$ | $CH_3$ | $n_D^{22}$ = 1,5613 |
| 4 | N | 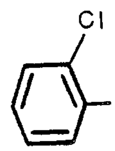 | 1 | $CH_3$ | H | H | $n_D^{22}$ = 1,5544 |
| 5 | N | 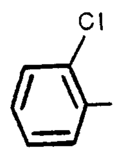 | 2 | H | $CH_3$ | $CH_3$ | $n_D^{22}$ = 1,5594 |
| 6 | N | 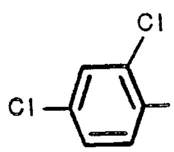 | 1 | H | $CH_3$ | $CH_3$ | $n_D^{22}$ = 1,5576 |
| 7 | N | 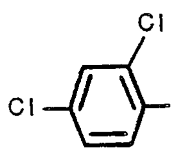 | 1 | $CH_3$ | H | H | $n_D^{22}$ = 1,5803 syn. Form |
| 8 | N | 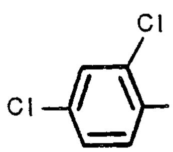 | 1 | $CH_3$ | H | H | $n_D^{22}$ = 1,5648 anti-Form |
| 9 | N | 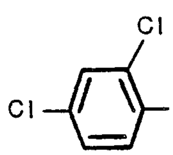 | 2 | H | $CH_3$ | $CH_3$ | $n_D^{22}$ = 1,5504 |
| 10 | CH | 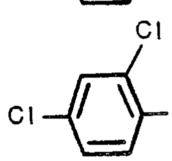 | 1 | H | H | H | $n_D^{22}$ = 1,5628 |

| Bsp. Nr. | Z | R | n | $R^1$ | $R^2$ | $R^3$ | Physikalische Daten |
|---|---|---|---|---|---|---|---|
| 11 | CH | 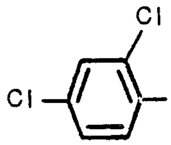 | 2 | H | H | H | $n_D^{22} = 1{,}5855$ |
| 12 | N | $(CH_3)_3C-$ | 2 | H | H | H | Kp 160°C/0,08 mbar |
| 13 | N | 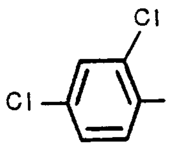 | 2 | H | H | H | $n_D^{22} = 1{,}5874$ |
| 14 | CH | 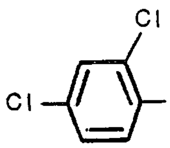 | 3 | H | H | H | *) δ = 4,25 ppm (t, 2H) |
| 15 | CH | 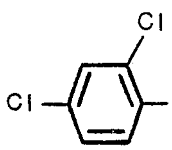 | 4 | H | H | H | *) δ = 4,25 ppm (t, 2H) |
| 16 | N | 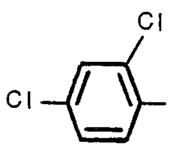 | 3 | H | H | H | *) δ = 4,30 ppm (t, 2H) |
| 17 | N | 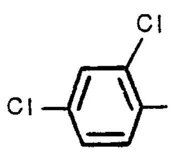 | 4 | H | H | H | *) δ = 4,2 ppm (t, 2H) |

$^1$H-NMR-Spektren ($CDCl_3$)
Die δ-Werte geben jeweils die chemische Verschiebung für die Gruppierung $—CH_2—O—N=$ an.

Verwendungsbeispiele

In den folgenden Verwendungsbeispielen werden die nachstehend angegebenen Substanzen als Vergleichsverbindungen eingesetzt:

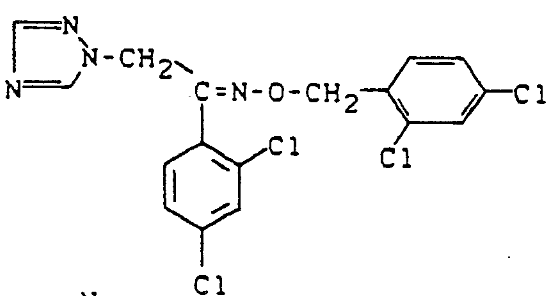 x $HNO_3$ (A)

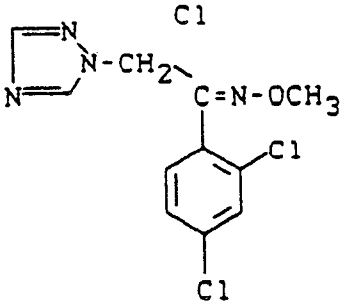 x $HNO_3$ (B)

Beispiel A

Sphaerotheca-Test (Gurke)/protektiv

Lösungsmittel: 4,7 Gewichtsteile Aceton

Emulgator: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentraat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23°C bis 24°C und bei einer relativen Luftfeuchtigkeit von ca. 75% im Gewächshaus aufgestellt.

10 Tage nach der Inokulation erfolgt die Auswertung.

In diesem Test zeigen die in den Beispielen 1, 2, 6, 7 und 8 offenbarten erfindungsgemäßen Stoffe eine bessere Wirksamkeit als Vergleichssubstanzen (A) und (B).

Beispiel B

Uromyces-Test (Buschbohne)/protektiv

Lösungsmittel: 4,7 Gewichtsteile Aceton

Emulgator: 0,3 Gewichsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wässrigen Uredosporensuspension des Bohnenrosterregers (Uromyces appendiculatus) inokuliert und verbleiben 1 Tag in einer dunklen Feuchtkammer bei 20°C bis 22°C und 100% relativer Luftfeuchtigkeit.

Die Pflanzen werden dann unter intensiver Belichtung für 9 Tage bei 20°C bis 22°C und einer relativen Luftfeuchtigkeit von 70% bis 80% im Gewächshaus aufgestellt.

10 Tage nach der Inokulation erfolgt die Auswertung.

In diesem Test zeigen die in den Beispielen 1, 2, 4, 6, 7, 8 und 12 offenbarten Stoffe eine wesentlich bessere Wirksamkeit als die Vergleichssubstanz (A).

**Patentansprüche**

1. Substituierte Cyclopropyl-oximether der Formel

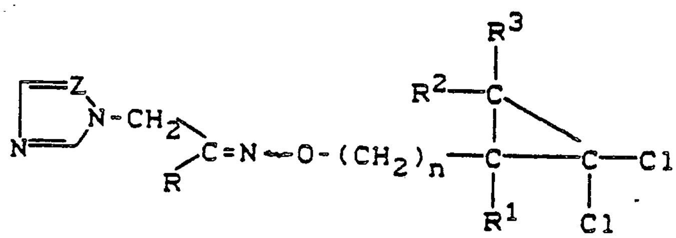

(I)

in welcher

Z für ein Stickstoffatom oder die CH-Gruppe steht,

R für die Gruppierungen

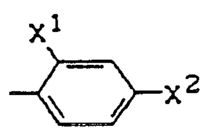 oder 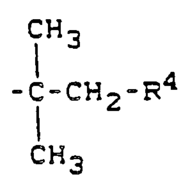 steht,

in welchen

$X^1$ und $X^2$ gleich oder verschieden sind und für Wasserstoff, Halogen oder Halogenalkyl stehen, wobei $X^1$ und $X^2$ nicht gleichzeitig für Wasserstoff stehen,

$R^4$ für Wasserstoff oder gegebenenfalls ein- bis dreifach, gleich oder verschieden substituiertes Phenyl steht, wobei als Substituenten Halogen und Alkyl mit 1 bis 4 Kohlenstoffatomen genannt seien,

$R^1$, $R^2$ und $R^3$ unabhängig voneinander für Wasserstoff, Methyl oder Chlor stehen und

n für ganze Zahlen von 1 bis 6 steht,

sowie deren Säureadditions-Salze und Metallsalz-Komplexe.

2. Substituierte Cyclopropyl-oximether der Formel (I) gemäß Anspruch 1, in denen
Z für ein Stickstoffatom oder die CH-Gruppe steht,
R für die Gruppierung

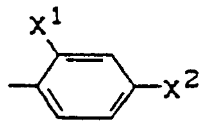 oder 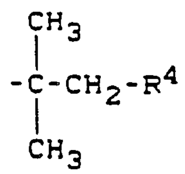 steht,

in welchen

$X^1$ und $X^2$ gleich oder verschieden sind und für Wasserstoff, Fluor, Chlor, Brom oder Halogenalkyl mit 1 oder 2 Kohlenstoffatomen und 1 bis 5 gleichen oder verschiedenen Halogenatomen stehen, wobei $X^1$ und $X^2$ nicht gleichzeitig für Wasserstoff stehen,

$R^4$ für Wasserstoff oder gegebenenfalls ein- oder zweifach, gleich oder verschieden substituiertes Phenyl steht, wobei als Substituenten Fluor, Chlor oder Methyl genannt seien,

$R^1$, $R^2$ und $R^3$ unabhängig voneinander für Wasserstoff, Methyl oder Chlor stehen und

n für ganze Zahlen von 1 bis 4 steht.

3. Substituierter Cyclopropyl-oximether der Formel

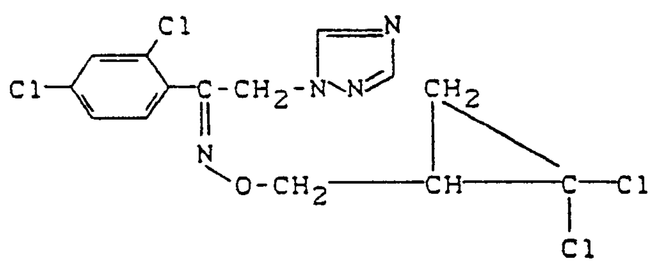

4. Substituierter Cyclopropyl-oximether der Formel

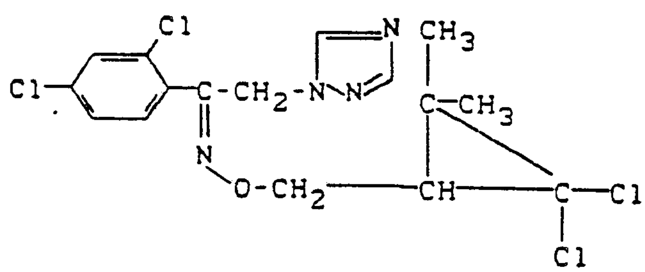

5. Verfahren zur Herstellung von substituierten Cyclopropyl-oximethern der Formel

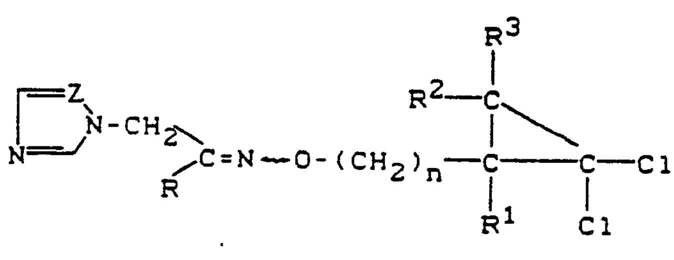 (I)

in welcher

Z für ein Stickstoffatom oder die CH-Gruppe steht,

R für die Gruppierungen

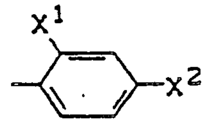 oder 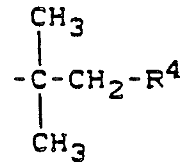 steht,

in welchen

$X^1$ und $X^2$ gleich oder verschieden sind und für Wasserstoff, Halogen oder Halogenalkyl stehen, wobei $X^1$ und $X^2$ nicht gleichzeitig für Wasserstoff stehen,

$R^4$ für Wasserstoff oder gegebenenfalls ein- bis dreifach, gleich oder verschieden substituiertes Phenyl steht, wobei als Substituenten Halogen und Alkyl mit 1 bis 4 Kohlenstoffatomen genannat seien,

$R^1$, $R^2$ und $R^3$ unabhängig voneinander für Wasserstoff, Methyl oder Chlor stehen und

n für ganze Zahlen von 1 bis 6 steht,

sowie von deren Säureadditions-Salzen und Metallsalz-Komplexen, dadurch gekennzeichnet, daß man Oxime der Formel

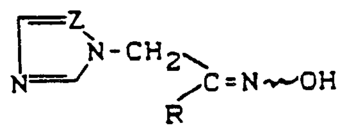

(II)

in welcher

Z und R die oben angegebene Bedeutung haben, mit Cyclopropan-Derivaten der Formel

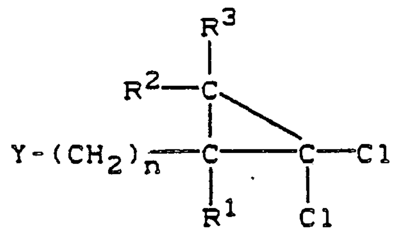

(III)

in welcher

$R^1$, $R^2$, $R^3$ und n die oben angegebene Bedeutung haben und

Y für Halogen oder p-Methyl-phenyl-sulfonyloxy steht, gegebenenfalls in Gegenwart einer starken Base und in Gegenwart eines Verdünnungsmittels umsetzt und gegebenenfalls an die so erhaltenen Verbindungen der Formel (I) eine Säure oder ein Metallsalz addiert.

6. Fungizide Mittel, gekennzeichnet durch einen Gehalt an mindestens einem substituierten Cyclopropyloximether der Formel (I) gemäß Anspruch 1 bzw. einem Säureadditions-Salz oder Metallsalz-Komplex eines substituierten Cyclopropyl-oximethers der Formel (I).

7. Verwendung von substituierten Cyclopropyl-oximethern der Formel (I) gemäß Anspruch 1 bzw. von deren Säureadditions-Salzen oder Metallsalz-Komplexen zur Bekämpfung von Pilzen.

8. Verfahren zur Bekämpfung von Pilzen, dadurch gekennzeichnet, daßm man subsituierte Cyclopropyl-oximether der Formel (I) gemäß Anspruch 1 bzw. deren Saüreadditions-Salze oder Metallsalz-Komplexe auf Pilze und/oder deren Lebensraum ausbringt.

9. Verfahren zur Herstellung von fungiziden Mitteln, dadurch gekennzeichnet, daß man substituierte Cyclopropyl-oximether der Formel (I) gemäß Anspruch 1 bzw. deren Säureadditions-Salze oder Metallsalz-Komplexe mit Streckmitteln und/oder oberflächenaktiven Stoffen vermischt.

**Revendications**

1. Cyclopropyl-oxime-éthers substitués de formule

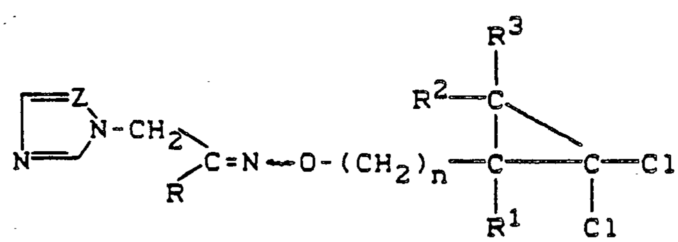

(I)

dans laquelle

Z est un atome d'azote ou le groupe CH,

R représente les groupements

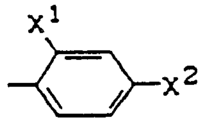

ou

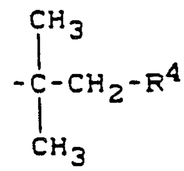

dans lesquels

$X^1$ et $X^2$ sont identiques ou différents et représentent l'hydrogène, un halogène ou un groupe halogénalkyle, $X^1$ et $X^2$ ne représentant pas simultanément de l'hydrogène,

$R^4$ est de l'hydrogène ou représente un groupe phényle portant éventuellement 1 à 3 substituants identiques ou différents, et on mentionne alors comme substituants un halogène et un groupe alkyle ayant 1 à 4 atomes de carbone,

$R^1$, $R^2$ et $R^3$ représentent indépendamment les uns des autres l'hydrogène, le groupe méthyle ou un atome de chlore et

n représente les nombres de 1 à 6,

ainsi que leurs sels d'addition d'acides et leurs complexes de sels métalliques.

2. Cyclopropyl-oxime-éthers substitués de formule (I) suivant la revendication 1, dans lesquels

Z est un atome d'azote ou le groupe CH,

R représente les groupements

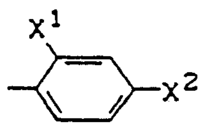

ou

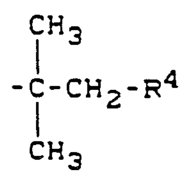

dans lesquels

$X^1$ et $X^2$ sont identiques ou différents et représentent l'hydrogène, le fluor, le chlore, le brome ou un groupe halogénalkyle ayant 1 ou 2 atomes de carbone et 1 à 5 atomes d'halogènes identiques ou différents, $X^1$ et $X^2$ ne représentant pas simultanément l'hydrogène,

$R^4$ représente l'hydrogène ou un groupe phényle portant éventuellement 1 ou 2 substituants identiques ou différents, et on mentionne alors comme substituants le fluor, le chlore ou le groupe méthyle,

$R^1$, $R^2$ et $R^3$ représentent indépendamment les uns des autres l'hydrogène, le groupe méthyle ou le chlore et

n représente des nombres entiers de 1 à 4.

3. Cyclopropyl-oxime-éthers substitués de formule

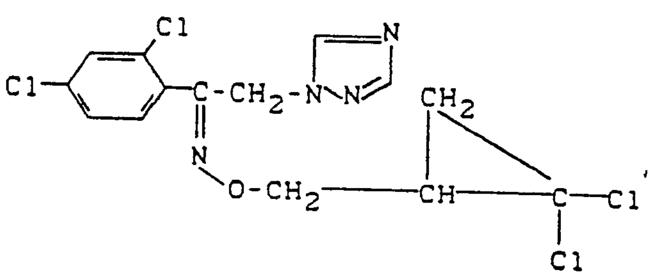

4. Cyclopropyl-oxime-éthers substitués de formule

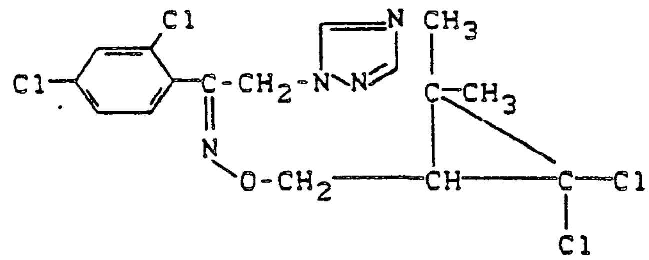

5. Procédé de préparation de cyclopropyl-oxime-éthers substitués de formule

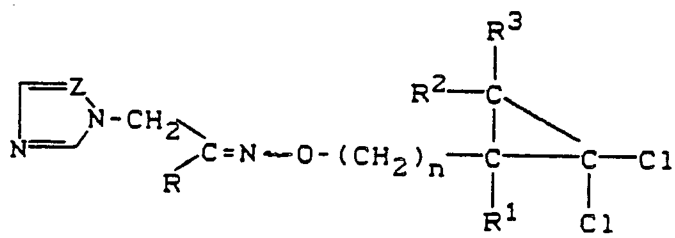

(I)

dans laquelle
Z représente un atome d'azote ou le groupe CH,
R représente les groupements

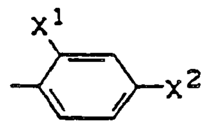

ou

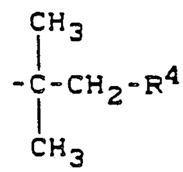

dans lesquels $X^1$ et $X^2$ sont identiques ou différents et représentent l'hydrogène, un halogène ou un groupe halogénalkyle, $X^1$ et $X^2$ ne représentant pas simultanément l'hydrogène,

$R^4$ représente l'hydrogène ou un groupe phényle portant éventuellement 1 à 3 substituants identiques ou différents, et on mentionne alors comme substituants un halogène et un groupe alkyle ayant 1 à 4 atomes de carbone,

$R^1$, $R^2$ et $R^3$ représentent indépendamment les uns des autres l'hydrogène, le groupe méthyle ou le chlore et

n représente les nombres entiers de 1 à 6, ainsi que leurs sels d'addition d'acides et leurs complexes de sels métalliques, caractérisé en ce qu'on fait réagir des oximes de formule

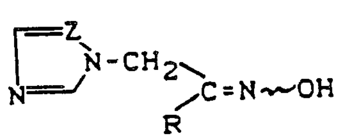

(II)

dans laquelle
Z et R ont la définition indiquée ci-dessus, avec des dérivés de cyclopropane de formule

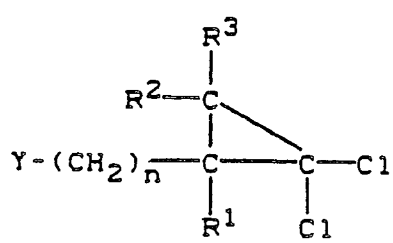

(III)

dans laquelle
$R^1$, $R^2$, $R^3$ et n ont les définitions indiquées ci-dessus et
Y représente un halogène ou le groupe p-méthylphényl-sulfonyloxy,
le cas échéant en présence d'une base forte et en présence d'un diluant et on additionne éventuellement un acide ou un sel métallique sur les composés de formule (I) ainsi obtenus.

6. Compositions fongicides, caractérisées par une teneur en au moins un cyclopropyl-oxime-éther substitué de formule (I) suivant la revendication 1 ou en un sel d'addition d'acide ou un complexe de sel métallique d'un cyclopropyl-oxime-éther substitué de formule (I).

7. Utilisation de cyclopropyl-oxime-éthers substitués de formule (I) suivant la revendication 1 ou de leurs sels d'addition d'acides ou de leurs complexes de sels métalliques pour combattre des champignons.

8. Procédé pour combattre des champignons, caractérisé en ce qu'on applique des cyclopropyl-oxime-ethers substitués de formule (I) suivant la revendication 1 ou leurs sels d'addition d'acides ou leurs complexes de sels métalliques sur des champignons et/ou sur leur milieu.

9. Procédé de préparation de compositions fongicides, caractérisé en ce qu'on mélange des cyclopropyl-oxime-éthers substitués de formule (I) suivant la revendication 1 ou leurs sels d'addition d'acides ou leurs complexes de sels métalliques avec des diluants et/ou des agents tensio-actifs.

**Claims**

1. Substituted cyclopropyl oxime ethers of the formula

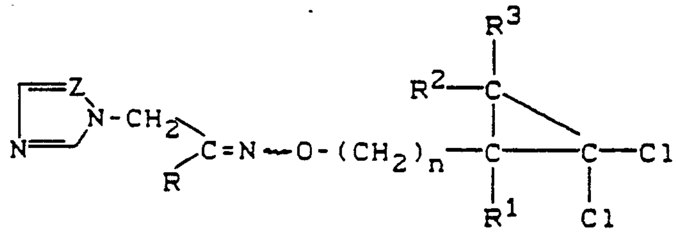

in which
Z represents a nitrogen atom or the CH group,
R represents the groupings

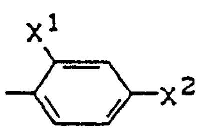

or

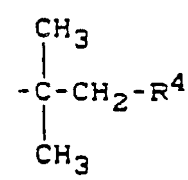

in which
$X^1$ and $X^2$ are identical or different and represent hydrogen, halogen or halogenoalkyl, $X^1$ and $X^2$ not simultaneously representing hydrogen, and $R^4$ represents hydrogen or phenyl which is optionally mono-, di- or trisubstituted by identical or different substituents, substituents which may be mentioned being halogen and alkyl with 1 to 4 carbon atoms,
$R^1$, $R^2$ and $R^3$ independently of one another represent hydrogen, methyl or chlorine and n represents integers from 1 to 6, and acid addition salts and metal salt complexes thereof.

2. Substituted cyclopropyl oxime ethers of the formula (I) according to Claim 1, in which
Z represents a nitrogen atom opr the CH group,
R represents the grouping

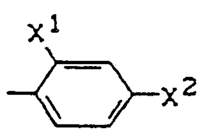

or

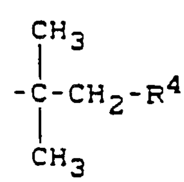

in which
$X^1$ and $X^2$ are identical or different and represent hydrogen, fluorine, chlorine, bromine or halogenoalkyl with 1 or 2 carbon atoms and 1 to 5 identical or different halogen atoms, $X^1$ and $X^2$ not simultaneously representing hydrogen, and
$R^4$ represents hydrogen or phenyl which is optionally mono- or disubstituted by identical or different

substituents, substituents which may be mentioned being fluorine, chlorine and methyl,

$R^1$, $R^2$ and $R^3$ independently of one another represent hydrogen, methyl or chlorine and n represents integers from 1 to 4.

3. Substituted cyclopropyl oxime ether of the formula

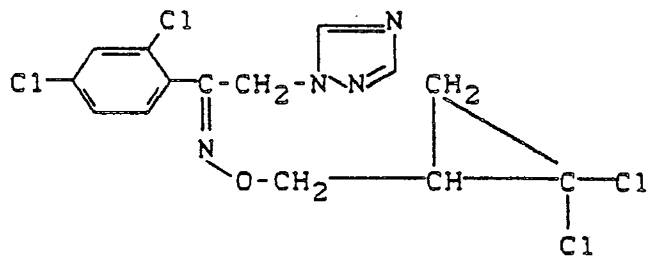

4. Substituted cyclopropyl oxime ether of the formula

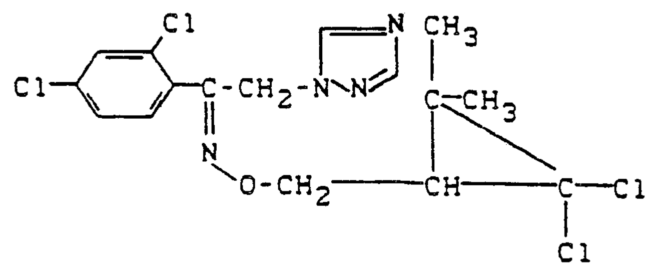

5. Process for the preparation of substituted cyclopropyl oxime ethers of the formula

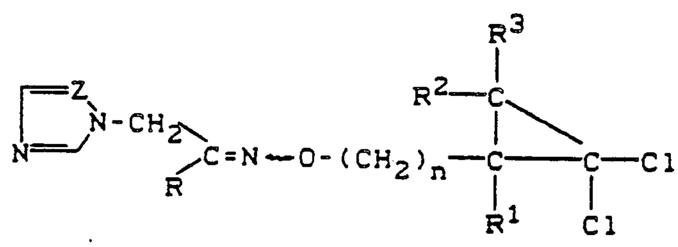

in which

Z represents a nitrogen atom or the CH group,

R represents optionally substituted phenyl, or represents the groupings

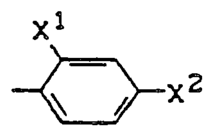 or 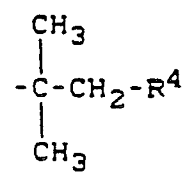

in which

$X^1$ and $X^2$ are identical or different and represent hydrogen, halogen or halogenoalkyl, $X^1$ and $X^2$ not simultaneously representing hydrogen, and $R^4$ represents hydrogen or phenyl which is optionally mono-, di- or trisubstituted by identical or different substituents, substituents which may be mentioned being halogen and alkyl with 1 to 4 carbon atoms,

$R^1$, $R^2$ and $R^3$ independently of one another represent hydrogen, methyl or chlorine and n represents integers from 1 to 6, and of acid addition salts and metal salt complexes thereof, characterized in that oximes of the formula

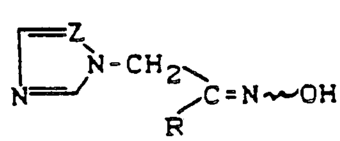

in which

Z and R have the abovementioned meaning, are reacted with cyclopropane derivatives of the formula

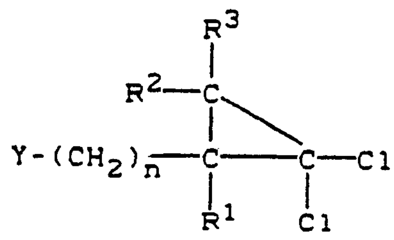

in which

$R^1$, $R^2$, $R^3$ and n have the abovementioned meaning and

Y represents halogen or p-methyl-phenyl-sulphonyloxy, if appropriate in the presence of a strong base and in the presence of a diluent, and, if appropriate, an acid or a metal salt is added onto the compounds of the formula (I) thus obtained.

6. Fungicidal agents, characterized in that they contaion at least one substituted cyclopropyl oxime ether of the formula (I) according to Claim 1 or an acid addition salt or metal salt complex of a substituted cyclopropyl oxime ether of the formula (I).

7. Use of substituted cyclopropyl oxime ethers of the formula (I) accordiong to Claim 1 or of acid addition salts or metal salt complexes thereof for combating fungi.

8. Method of combating fungi, characterized in that substituted cyclopropyl oxime ethers of the formula (I) according to Claim 1 and acid addition salts or metal salt complexes thereof are applied to fungi and/or their environment.

9. Process for the preparation of fungicidal agents, characterized in that substituted cyclopropyl oxime ethers of the formula (I) according to Claim 1 or acid addition salts or metal salt complexes thereof are mixed with extenders and/or surface-active substances.